# EUROPEAN PATENT APPLICATION

(11) **EP 4 470 515 A1**
(43) Date of publication of application: **04.12.2024**
(21) Application number: 23752938.3
(22) Date of filing: 09.02.2023
(51) Int. Cl.: A61F 13/49, A61F 13/496

(54) **DISPOSABLE WEARABLE ARTICLE**

(30) Priority: 14.02.2022 JP 2022020126
(71) Applicant: Zuiko Corporation, Ibaraki-shi Osaka 5670082 (JP)
(72) Inventor: MATSUO Maki, Ibaraki-shi, Osaka 567-0082 (JP)
(74) Representative: Hasegawa, Kan
(86) International application number: PCT/JP2023/004368
(87) International publication number: WO 2023/153474

(57) **Abstract**

A pants-type disposable wearable article where front and back waist portions include plural long and thin elastic members that extend in a waist direction and are arranged parallel to each other and a pair of sheets that sandwich the elastic members and retain the elastic members with plural fastening portions that fasten the elastic members intermittently in the waist direction, side seals include plural dot-like welded portions that are arranged in a studded manner and weld and bond the front and back waist portions to each other, the plural welded portions include, relative to one elastic member, an upper welded portion and a lower welded portion that are spaced apart in an upper-lower direction orthogonal to the waist direction, and a spacing in the upper-lower direction between the upper welded portion and the lower welded portion is larger than a first width of the elastic members in a stretched state and smaller than a second width of the elastic members in a contracted state.

## Description

### TECHNICAL FIELD

The present invention relates to what is called a pants-type disposable wearable article.

### BACKGROUND ART

Patent Document 1, listed below, discloses a method of manufacturing a wearable article. In this conventional manufacturing method, rubber threads in a stretched state are sandwiched between two webs (nonwoven fabrics), the webs and the rubber threads (elastic members) are fastened intermittently in a contraction direction to form a stretchable sheet, the stretchable sheet is used as at least one of a front body or back body (waist portion) of a wearable article, and both edge portions of the front body and the back body are side-sealed (welded) to form a waist opening and leg openings.

### CITATION LIST

### Patent Literature

Patent Document 1: WO 2020/235495 (Front Page)

### SUMMARY OF INVENTION

In this kind of pants-type wearable article, if the strength with which the rubber threads are fastened to the webs is not sufficient, the end portions of the rubber threads that have been cut at the both edge portions that have been side-sealed end up significantly moving (snapping back), giving rise to the problems of a diminished visual appearance and a decrease in fit.

It is an object of the present invention to provide a disposable wearable article that can prevent elastic members from significantly snapping back at side seals to prevent a reduction in attractiveness and a decrease in fit.

The present invention is a pants-type disposable wearable article having front and back waist portions 31, 32 whose edge portions are bonded to each other by a pair of side seals 50,
wherein
the front and back waist portions 31, 32 include
   plural long and thin elastic members F configured to extend in a waist direction X and are arranged parallel to each other and
   a pair of sheets 1, 2 that sandwich the elastic members F and retain the elastic members F with plural fastening portions 4 that fasten the elastic members F intermittently in the waist direction X,
the side seals 50 include plural dot-like welded portions 5 that are arranged in a studded manner and weld and bond the front and back waist portions 31, 32 to each other,
the plural welded portions 5 include, relative to at least one elastic member F of the elastic members F, at least one upper welded portion 5a and at least one lower welded portion 5b that are spaced apart in an upper-lower direction Y orthogonal to the waist direction X, and
a spacing ΔS in the upper-lower direction Y between the upper welded portion 5a and the lower welded portion 5b is larger than a first width F1 of the elastic members F in a stretched state and smaller than a second width F2 of the elastic members F in a contracted state.

According to the present invention, the spacing ΔS in the upper-lower direction Y between the upper welded portion 5a and the lower welded portion 5b is smaller than the second width F2 of the elastic members F in a contracted state, and in this contracted state upper and lower pairs of welded portions nip the elastic members F from above and below. For that reason, snapback in which the elastic members F move in the waist direction X can be prevented. Consequently, there is no concern of a deterioration in the outward appearance of the wearable article N or a decrease in fit.

The side seals in which the dot-like welded portions 5 are arranged in a studded manner have a small welded area, and for that reason both edge portions of the waist portions of the pants-type wearable article N are less likely to become stiff, resulting in an improved feel.

It will be noted that the spacing ΔS is larger than the first width F1 of the elastic members F in a stretched state, and the sheets can be welded together.

In the present invention, it is preferred that the sheets be thermoplastic nonwoven fabrics (webs) in which thermoplastic fibers are layered.

The long and thin elastic members may be linear or band-like. For example, the elastic members may be multistrand members in which plural rubber threads (fibrous elastic bodies) are gathered in a bundle. Examples of the material of the rubber threads include polyurethane.

In the present invention, the "upper-lower direction Y" means the upper-lower direction along a vertical direction when a wearer is wearing the wearable article N in a standing position.

Furthermore, in the present invention, "upper side" means the upper side surface of one elastic member F and "lower side" means the lower side surface of that elastic member F.

### BRIEF DESCRIPTION OF DRAWINGS

FIG. 1A is a front view showing an example of a wearable article manufactured by a manufacturing method of the present invention, and FIG. 1B is a transverse sectional view.
FIG. 2 is an enlarged front view of the same wearable article.
FIG. 3 is an enlarged front view showing elastic members, fastening portions, and welded portions.
FIG. 4 is a front view showing a manufacturing process of the wearable article.
FIG. 5 is a front view showing the elastic members and the fastening portions in a stretched state.
FIG. 6 is a front view showing another manufacturing process of the wearable article.
FIG. 7 is an enlarged front view showing the elastic members, the fastening portions, and the welded portions in a stretched state.

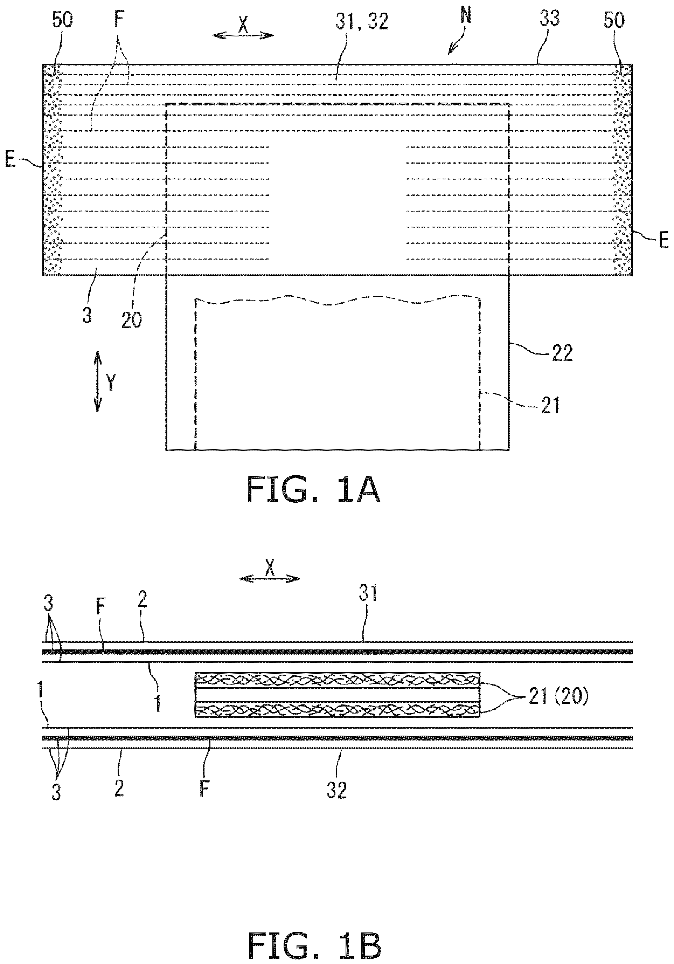

It will be noted that in FIG. 1A, FIG. 2, FIG. 4, and FIG. 6, thin and short dashed lines represent a wearable article N while thick and long dashed lines represent an absorbent body 20.

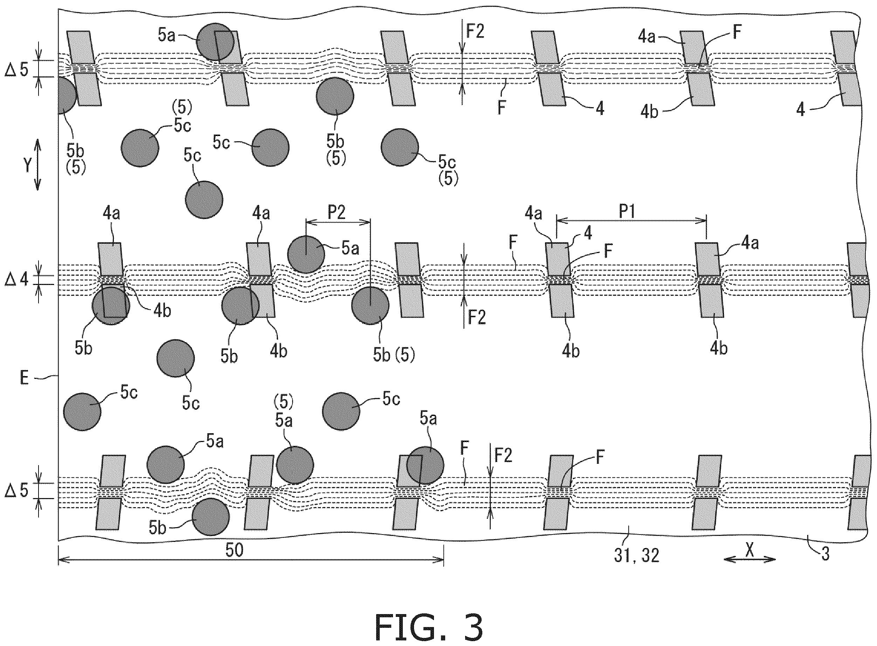

Furthermore, in FIG. 3, FIG. 5, and FIG. 7, fastening portions 4 are colored in light gray, welded portions 5 are colored in dark gray, and elastic members F are depicted with dashed lines.

### DESCRIPTION OF EMBODIMENTS

The present invention will become more clearly understood from the following description of preferred embodiments taken in conjunction with the accompanying drawings. However, the embodiments and the drawings are merely for the purposes of illustration and explanation and should not be utilized to define the scope of the present invention. The scope of the present invention shall be defined only by the claims. In the accompanying drawings, like parts numbers in multiple views refer to the same or corresponding parts.

The structure of a wearable article pertaining to an embodiment of the present invention will be described below in accordance with the drawings.

FIG. 1 to FIG. 7 show one embodiment.

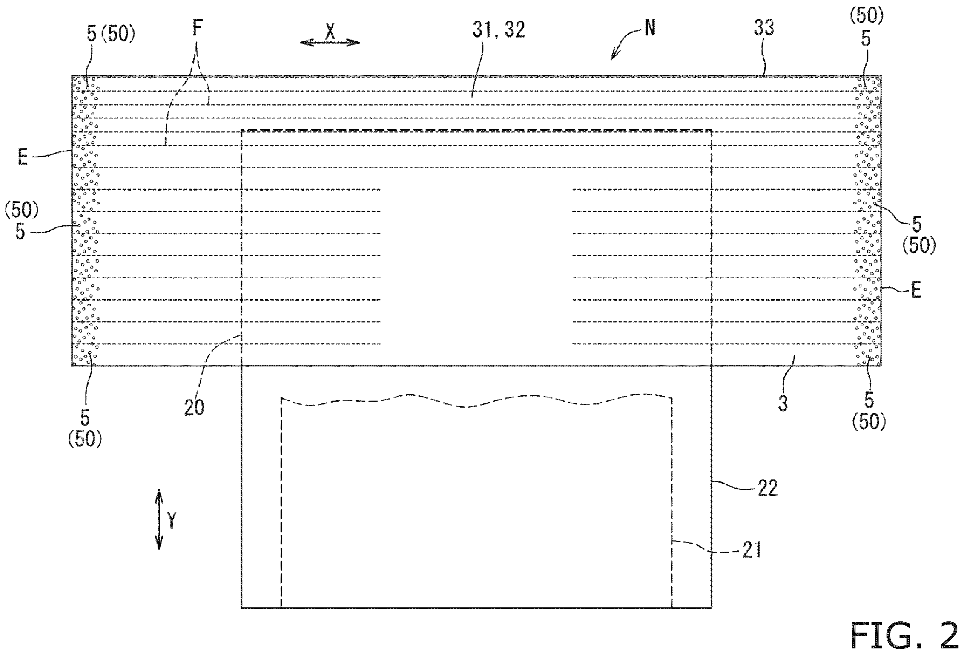

As shown in FIG. 1A and FIG. 2, the wearable article of the present embodiment 1 has a bilaterally symmetrical shape and structure, and includes an absorbent body 20 and front and back waist portions 31, 32. The absorbent body 20 has a front torso portion that covers the front torso of the wearer and extends in a waist direction X, a back torso portion that covers the back torso of the wearer and extends in the waist direction X, and a crotch portion 22 that covers the crotch area between both torso portions.

The crotch portion 22 extends in a longitudinal direction Y orthogonal to the waist direction X. The absorbent body 20 configures part of or all the crotch portion 22.

The waist portions 31, 32 of FIG. 1B are, as described later, formed by sandwiching plural long and thin elastic members F between a first web (sheet) 1 and a second web (sheet) 2.

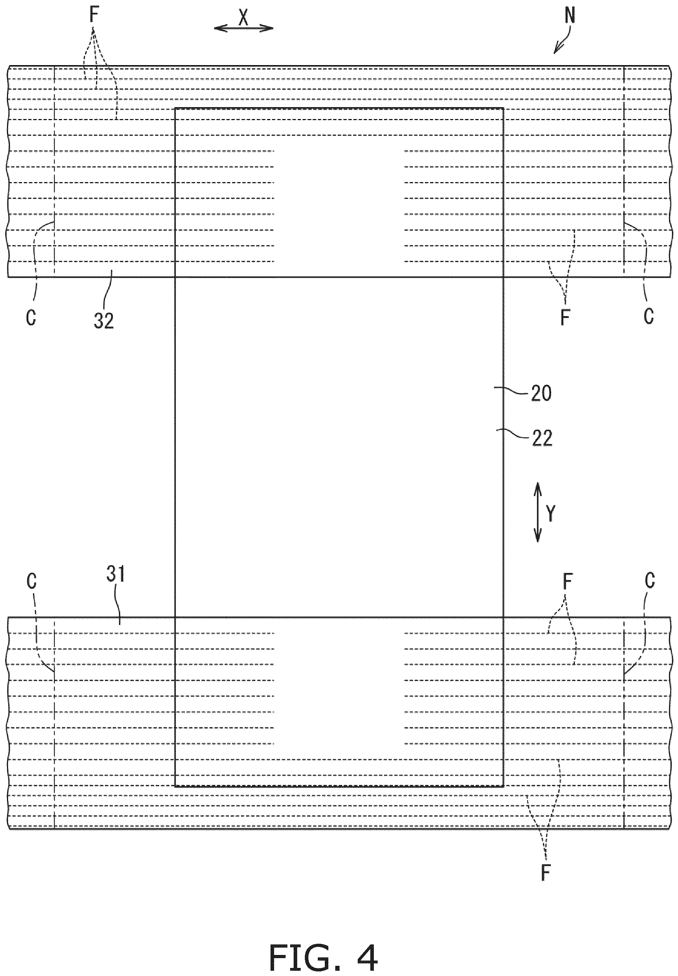

As in FIG. 1A, in the state of the finished product, the crotch portion 22 is folded in two at a line parallel to the waist direction X. Because of this, edge portions E in the waist direction X of the front and back waist portions 31, 32 of FIG. 4 overlap each other as in FIG. 1. The wearable article is a pants-type, and the edge portions in the waist direction X of the front waist portion 31 and the back waist portion 32 are welded to each other.

The absorbent body 20 of FIG. 2 is provided with an absorbent core 21. The absorbent core 21 absorbs bodily fluids. The absorbent core 21 is sandwiched between a topsheet and a backsheet.

The topsheet comprises a thin nonwoven fabric permeable to liquid and covers the skin side of the absorbent core 21. Cuffs not shown in the drawings may be provided on the topsheet.

The backsheet covers the non-skin side of the absorbent core 21 and comprises a resin sheet impermeable to liquid. The waist portions 31, 32 of FIG. 2 are adhered to edge portions in the longitudinal direction Y of the absorbent body 20.

In FIG. 2, the waist portions 31, 32 project in the waist direction X from the absorbent body 20. In other words, the waist portions 31, 32 project in the waist direction X beyond the crotch portion 22 and extend out in the waist direction X from both edge portions (edge portions in the waist direction) of the absorbent body 20.

Each of the waist portions 31, 32 is provided with elastic members F for fitting the wearable article to the wearer. As the elastic members F, for example, materials including plural rubber threads or flat rubber bands or thermoplastic resins can be employed. Furthermore, the elastic members F may be deactivated (put in a state in which no contractile force acts on them) in the parts overlapping the absorbent body 20. The elastic members F may extend in the waist direction X parallel to each other.

In the absorbent body 20, leg portions that are narrowed along the legs of the wearer may be formed.

The absorbent body 20 of FIG. 1B is adhered to skin surfaces of the waist portions 31, 32.

In the present specification, "skin surfaces" refers to surfaces facing the skin of the wearer when the wearer is wearing the wearable article and "non-skin surfaces" refers to surfaces opposite the skin surfaces.

Next, further details about the wearable article will be described.

As shown in FIG. 1A, wearable article N is a pants-type disposable wearable article having front and back waist portions 31, 32 whose edge portions are bonded to each other by a pair of side seals 50.

The front and back waist portions 31, 32 (FIG. 1B) include, as in FIG. 2, plural long and thin elastic members F that may extend in the waist direction X and are arranged parallel to each other and a pair of webs 1, 2 (FIG. 1B) that sandwich the elastic members F of FIG. 1B and retain the elastic members F with plural fastening portions 4 that fasten the elastic members F intermittently in the waist direction X as in FIG. 3.

In FIG. 3 the side seals 50 include plural dot-like welded portions 5 arranged in a studded manner, and the welded portions 5 weld and bond the front and back waist portions 31, 32 (FIG. 1 B) to each other. The side seals 50 of FIG. 2 interconnect the front and back waist portions 31, 32 to create a loop, forming a waist opening 33 that opens upward and downward. The side seals 50 are provided in areas of edge portions close to edges E of the waist portions 31, 32.

Next, before more detailed description of the welded portions 5 of FIG. 3, the fastening portions 4 will be described in conjunction with part of the manufacturing process of the wearable article N.

As shown in FIG. 4, the wearable article N has the front and back waist portions 31, 32 bridged by the absorbent body 20. The waist portions 31, 32 are conveyed in a state in which the pair of continuous webs 1, 2 and numerous elastic members F are stretched in the waist direction X which is the conveyance direction.

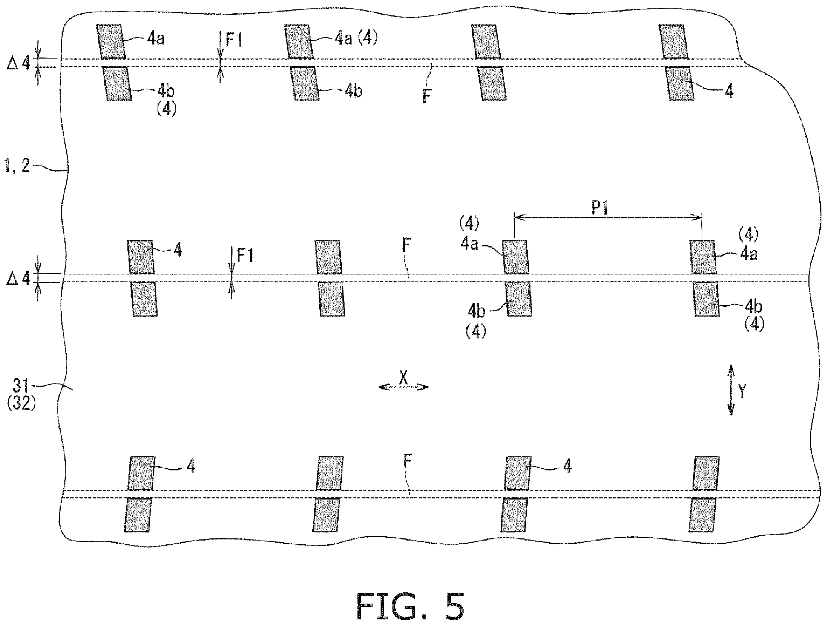

In this manufacturing process, the elastic members F in the stretched state are thin as in FIG. 5.

As shown in an enlargement in FIG. 5, the pair of webs 1, 2 are welded to each other on upper sides and lower sides of the elastic members F to retain the elastic members F in the pair of webs 1, 2.

Each of the fastening portions 4 includes an upper fastening portion 4a at which, relative to one elastic member F, the pair of webs 1, 2 are welded together on the upper side of the elastic member F and a lower fastening portion 4b at which, relative to the one elastic member F, the pair of webs 1, 2 are welded together on the lower side of the elastic member F.

A spacing Δ4 in the upper-lower direction Y between the upper fastening portion 4a and the lower fastening portion 4b is larger than a first width F1 of the elastic members F in a stretched state as in FIG. 5 and smaller than a second width F2 of the elastic members F in a contracted state before being worn as in FIG. 3. Because of this, the elastic members F can be fastened by the fastening portions 4.

It will be noted that in the following description reference sign "4" is added when collectively referring to the fastening portions.

Next, details about the welded portions 5 will be described in conjunction with the fastening portions 4.

As shown in FIG. 3, the plural welded portions 5 include, relative to at least one elastic member F of the elastic members F, at least one upper welded portion 5a and at least one lower welded portion 5b that are spaced apart in the upper-lower direction Y orthogonal to the waist direction X.

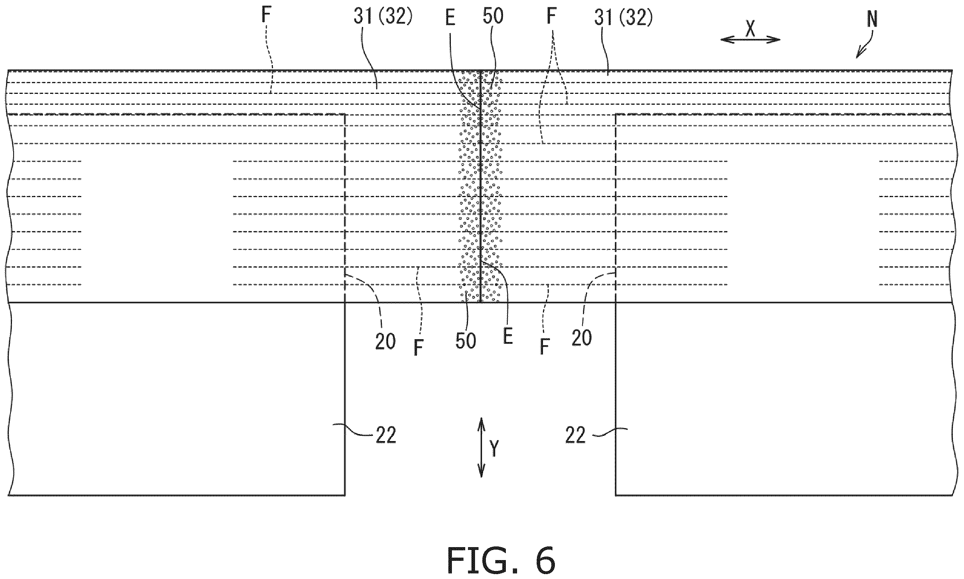

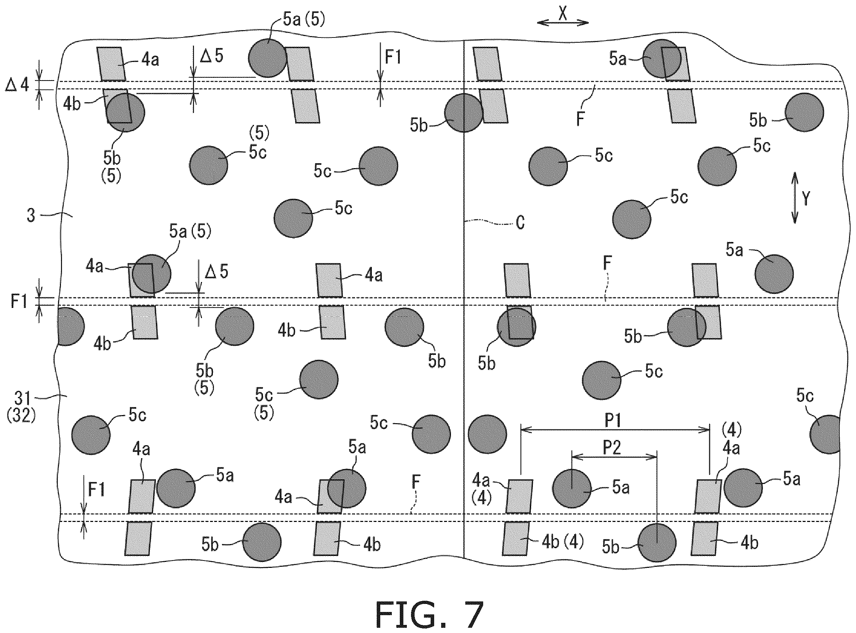

In the manufacturing process of FIG. 6, the elastic members F are in a stretched state and are thin as in FIG. 7. A spacing ΔS in the upper-lower direction Y between the upper welded portion 5a and the lower welded portion 5b is larger than the first width F1 of the elastic members F in a stretched state and smaller than the second width F2 of the elastic members F in a contracted state of FIG. 3. Because of this, the elastic members F are engaged with the welded portions 5.

That is, the relationship between the spacing ΔS and the first width F1 and second width F2 of the elastic members F is set as described above, and the upper welded portion 5a is in contact with a side surface on the upper side of the one elastic member F and the lower welded portion 5b is in contact with a side surface on the lower side of that elastic member F, whereby the upper and lower welded portions 5a, 5b are engaged in the waist direction X with the one elastic member F.

For that reason, just after the waist portions 31, 32 in the continuous and stretched state of FIG. 6 and FIG. 7 are cut along a virtual cutting line C, the welded portions 5 engage in the waist direction X with the contracted and thickened elastic members F of FIG. 3 to prevent snapback of the elastic members F.

As in the present example of FIG. 3, in the side-sealed edge portions, one of the at least one upper or lower welded portion 5a, 5b may be provided as a plurality spaced apart from each other in the waist direction X.

Furthermore, the other of the at least one upper or lower welded portion 5a, 5b may be provided in a position between, in the waist direction X, the upper or lower welded portions 5a, 5b provided as a plurality spaced apart in the waist direction X.

In FIG. 3, the plural fastening portions 4 are provided spaced apart from each other in the waist direction X at a first pitch P1 relative to the at least one elastic member F. In contrast, the plural upper or lower welded portions 5a, 5b are provided at a second pitch P2 in the waist direction X. The second pitch P2 is smaller than the first pitch P1 in the case of the present example.

In the case of the present example of FIG. 3, the upper welded portion 5a and the lower welded portion 5b are respectively larger in area than the upper fastening portion 4a and larger in area than the lower fastening portion 4b. It will be noted that the fastening portions 4 may be larger than the welded portions 5. For example, in the case of providing the fastening portions 4 in long lines extending in the upper-lower direction Y, the area of each fastening portion 4 becomes larger than the area of each welded portion 5.

As shown in FIG. 3, in the present example, the upper welded portion 5a and the lower welded portion 5b are provided for each of the plural elastic members F. In a case where the upper welded portion 5a and the lower welded portion 5b are provided in pluralities relative to one elastic member F, each welded portion 5a or 5b may be provided in the same position in the longitudinal direction to configure a welded portion column forming a column.

Furthermore, the plural welded portions 5 may include plural intermediate welded portions 5c. Each intermediate welded portion 5c is disposed in a position in which it is not in contact with either of the elastic members F of mutually adjacent elastic members F of the plural elastic members F.

The specific embodiment described above includes inventions having the following configurations.

As a preferred embodiment, the relationship between the spacing ΔS and the first width F1 and the second width F2 of the elastic members F is set as described above, and
the upper welded portion 5a is in contact with a side surface on the upper side of the one elastic member F and the lower welded portion 5b is in contact with a side surface on the lower side of that elastic member F,
whereby the upper and lower welded portions 5a, 5b are engaged in the waist direction X with the one elastic member F.

In this case, each of the welded portions 5 is engaged in the waist direction X with the upper side and the lower side of the one elastic member F, and for that reason the elastic member F hooks onto the upper and lower pair of welded portions 5 so that snapback of the elastic member F can be prevented.

More preferably, one of the at least one upper or lower welded portion 5a, 5b is provided as a plurality spaced apart from each other in the waist direction X.

In this case, the upper or lower welded portions 5 provided as a plurality cause the elastic member F in a contracted state to bend in plural places, and for that reason the effect of preventing snapback is enhanced.

More preferably, the other of the at least one upper or lower welded portion 5a, 5b is provided in a position between, in the waist direction X, the upper or lower welded portions 5a, 5b provided as a plurality spaced apart in the waist direction X.

In this case, the welded portions 5 cause the elastic member F to bend in three places, and for that reason the effect of preventing snapback is further enhanced.

In the present invention, the pair of sheets 1, 2 may be welded to each other on upper sides and lower sides of the elastic members F, whereby the fastening portions 4 retain the elastic members F in the pair of sheets 1, 2.

It will be noted that the fastening portions 4 may also be formed by adhesion rather than welding.

In a more preferred embodiment, the plural fastening portions 4 adjacent to each other in the waist direction X are provided spaced apart from each other in the waist direction X at a first pitch P1 relative to the at least one elastic member F,
the plural upper or lower welded portions 5a, 5b adjacent to each other in the waist direction X are provided at a second pitch P2 in the waist direction X, and
the second pitch P2 is smaller than the first pitch P1.

In this case, the second pitch P2 of the welded portions 5 that engage with the elastic member F is small, and for that reason the effect of preventing snapback is further enhanced.

In the present invention, each of the fastening portions 4 may include an upper fastening portion 4a at which, relative to the one elastic member F, the pair of sheets 1, 2 are welded together on the upper side of the elastic member F and a lower fastening portion 4b at which, relative to the one elastic member F, the pair of sheets 1, 2 are welded together on the lower side of the elastic member F.

In a preferred embodiment, the upper welded portion 5a and lower welded portion 5b are respectively larger in area than the upper fastening portion 4a and larger in area than the lower fastening portion 4b.

Because the area of each welded portion 5 is larger than the area of each fastening portion 4, a reliable joint strength is easily obtained for the side seals and a flexible feel is obtained for the waist portions overall because the fastening portions 4 are small.

In the present invention, a spacing Δ4 in the upper-lower direction Y between the upper fastening portion 4a and the lower fastening portion 4b may be larger than the first width F1 of the elastic members F in a stretched state and smaller than the second width F2 of the elastic members F in a contracted state.

In a case where the spacing Δ4 is set in this way, it is easy to obtain strength with which to fasten the elastic members F with the upper and lower fastening portions 4.

Preferably, the upper welded portion 5a and the lower welded portion 5b are provided for each of the plural elastic members F.

In this case, snapback of each elastic member F can be prevented.

More preferably, the plural welded portions 5 include plural intermediate welded portions 5c, and
each intermediate welded portion 5c is disposed in a position in which it is not in contact with either of the elastic members F of mutually adjacent elastic members F of the plural elastic members F.

In this case, the intermediate welded portions 5c join the front and back waist portions 31, 32 between the elastic member F and the elastic member F, which enhances the reliability of the joint by the side seals.

The features described and/or illustrated in association with one embodiment or preferred embodiments can be used in the same or similar form in one or more other embodiments and/or in combination with, or instead of, other embodiments.

While preferred embodiments have been described above with reference to the drawings, a variety of obvious changes and modifications will readily occur to those skilled in the art upon reading the present specification.

For example, each of the welded portions may be quadrilateral or elliptical rather than circular in shape. Furthermore, the shapes and sizes of each of the welded portions need not be uniform.

Furthermore, the diaper may be a recloseable pants-type diaper, and in this case the absorbent body may be a crotch member without an absorber.

Furthermore, the fastening portions may be continuous in the upper-lower direction, and the sheets and the elastic members may also be welded at the fastening portions.

Consequently, such changes and modifications are to be interpreted as being within the scope of the present invention as defined by the claims.

### INDUSTRIAL APPLICABILITY

The present invention can be applied mainly to pants-type disposable wearable articles.

### REFERENCE SIGNS LIST

1, 2: Sheets (Webs)
20: Absorbent Body, 22: Crotch Portion, 21: Absorbent Core
3: Stretchable Sheets, 31: Front Waist Portion, 32: Back Waist Portion, 33: Waist Opening
4: Fastening Portions, 4a: Upper Fastening Portions, 4b: Lower Fastening Portions
50: Side Seals
5: Welded Portions, 5a: Upper Welded Portions, 5b: Lower Welded Portions, 5c: Intermediate Welded Portions
C: Cutting Line
E: Edges
F: Elastic Members, N: Wearable Article, F1: First Width, F2: Second Width
P1: First Pitch, P2: Second Pitch
X: Waist Direction (Lengthwise Direction), Y: Upper-lower Direction
Δ4, ΔS: Spacings

## Claims

1. A pants-type disposable wearable article having front and back waist portions 31, 32 whose edge portions are bonded to each other by a pair of side seals 50,
wherein
the front and back waist portions 31, 32 include
plural long and thin elastic members F configured to extend in a waist direction X and are arranged parallel to each other and
a pair of sheets 1, 2 that sandwich the elastic members F and retain the elastic members F with plural fastening portions 4 that fasten the elastic members F intermittently in the waist direction X,
the side seals 50 include plural dot-like welded portions 5 that are arranged in a studded manner and weld and bond the front and back waist portions 31, 32 to each other,
the plural welded portions 5 include, relative to at least one elastic member F of the elastic members F, at least one upper welded portion 5a and at least one lower welded portion 5b that are spaced apart in an upper-lower direction Y orthogonal to the waist direction X, and
a spacing ΔS in the upper-lower direction Y between the upper welded portion 5a and the lower welded portion 5b is larger than a first width F1 of the elastic members F in a stretched state and smaller than a second width F2 of the elastic members F in a contracted state.

2. The disposable wearable article of claim 1, wherein
the relationship between the spacing ΔS and the first width F1 and the second width F2 of the elastic members F is set as described above, and
the upper welded portion 5a is in contact with a side surface on an upper side of the one elastic member F and the lower welded portion 5b is in contact with a side surface on a lower side of that elastic member F,
whereby the upper and lower welded portions 5a, 5b are engaged in the waist direction X with the one elastic member F.

3. The disposable wearable article of claim 2, wherein one of the at least one upper or lower welded portion 5a, 5b is provided as a plurality spaced apart from each other in the waist direction X.

4. The disposable wearable article of claim 3, wherein the other of the at least one upper or lower welded portion 5a, 5b is provided in a position between, in the waist direction X, the upper or lower welded portions 5a, 5b provided as a plurality spaced apart in the waist direction X.

5. The disposable wearable article of claim 4, wherein the pair of sheets 1, 2 are welded to each other on upper sides and lower sides of the elastic members F, whereby the fastening portions 4 retain the elastic members F in the pair of sheets 1, 2.

6. The disposable wearable article of claim 5, wherein
the plural fastening portions 4 adjacent to each other in the waist direction X are provided spaced apart from each other in the waist direction X at a first pitch P1 relative to the at least one elastic member F,
the plural upper or lower welded portions 5a, 5b adjacent to each other in the waist direction X are provided at a second pitch P2 in the waist direction X, and
the second pitch P2 is smaller than the first pitch P1.

7. The disposable wearable article of claim 5, wherein each of the fastening portions 4 includes an upper fastening portion 4a at which, relative to the one elastic member F, the pair of sheets 1, 2 are welded together on an upper side of the elastic member F and a lower fastening portion 4b at which, relative to the one elastic member F, the pair of sheets 1, 2 are welded together on a lower side of the elastic member F.

8. The disposable wearable article of claim 7, wherein the upper welded portion 5a and lower welded portion 5b are respectively larger in area than the upper fastening portion 4a and larger in area than the lower fastening portion 4b.

9. The disposable wearable article of claim 7, wherein a spacing Δ4 in the upper-lower direction Y between the upper fastening portion 4a and the lower fastening portion 4b is larger than the first width F 1 of the elastic members F in a stretched state and smaller than the second width F2 of the elastic members F in a contracted state.

10. The disposable wearable article of claim 8, wherein a spacing Δ4 in the upper-lower direction Y between the upper fastening portion 4a and the lower fastening portion 4b is larger than the first width F1 of the elastic members F in a stretched state and smaller than the second width F2 of the elastic members F in a contracted state.

11. The disposable wearable article of any one of claims 1 to 10, wherein the upper welded portion 5a and the lower welded portion 5b are provided for each of the plural elastic members F.

12. The disposable wearable article of claim 11, wherein
the plural welded portions 5 include plural intermediate welded portions 5c, and
each intermediate welded portion 5c is disposed in a position in which it is not contact with either of the elastic members F of mutually adjacent elastic members F among the plural elastic members F.
